Europäisches Patentamt

(19) European Patent Office

Office européen des brevets

(11) Numéro de publication: **0 109 320 B1**

## (12) FASCICULE DE BREVET EUROPEEN

(45) Date de publication du fascicule du brevet: **25.06.86**

(21) Numéro de dépôt: **83402073.7**

(22) Date de dépôt: **25.10.83**

(51) Int. Cl.⁴: **A 61 K 9/26**, A 61 K 31/52

---

(54) **Comprimés de théophylline à libération controlée et leur procédé de fabrication.**

---

(30) Priorité: **03.11.82 FR 8218407**

(43) Date de publication de la demande:
**23.05.84 Bulletin 84/21**

(45) Mention de la délivrance du brevet:
**25.06.86 Bulletin 86/26**

(84) Etats contractants désignés:
**AT BE CH DE GB IT LI LU NL SE**

(56) Documents cités:
**AT - A - 347 039**
**FR - A - 2 070 139**
**FR - A - 2 120 101**
**US - A - 4 259 314**
**US - A - 4 261 970**

**CHEMICAL ABSTRACTS, vol. 96, no. 1, janvier 1982, page 336, no. 11678k, Columbus, Ohio, US**
**HAGERS HANDBUCH DER PHARMAZEUTISCHEN PRAXIS, vol. 7, partie A, édition 4, 1971, pages 718-723, Springer-Verlag, Berlin, DE**

**Le dossier contient des informations techniques présentées postérieurement au dépôt de la demande et ne figurant pas dans le présent fascicule.**

(73) Titulaire: **P.F. MEDICAMENT, 125, Rue de la Faisanderie, F-75116 Paris (FR)**

(72) Inventeur: **Bougaret, Joel, 22 rue de Saint Ouen, F-75018 Paris (FR)**
Inventeur: **Darbeau, Daniel, 53 rue Ganneron, F-75018 Paris (FR)**

(74) Mandataire: **Martin, Jean-Jacques et al, Cabinet REGIMBEAU 26, Avenue Kléber, F-75116 Paris (FR)**

---

ACTORUM AG

## Description

La présente invention concerne de nouveaux comprimés pharmaceutiques constitués par une matrice polymère hydrophile permettant une libération contrôlée et constante d'une dose thérapeutique unitaire de théophylline pendant un intervalle de temps d'environ 12 heures, ainsi que leur procédé de fabrication.

La fabrication de ce type de matrices hydrophiles consiste généralement à mélanger un principe actif relativement soluble avec un agent gélifiant de viscosité élevée, par exemple constitué par un polymère ou une gomme hydrophile non digestible. L'obtention de ces matrices fait appel à la compression directe ou à la granulation sèche ou humide, on peut donc y adjoindre suivant les cas divers autres excipients.

De nombreux agents viscosifiants sont mentionnés dans la littérature, depuis les polymères naturels tels que les alginates et les pectines, jusqu'aux dérivés synthétiques ou semi-synthétiques.

En fait, si grand nombre de travaux se sont succédés pour décrire les principes de base, les recherches orientées vers la mise en évidence de l'influence de la formulation, des facteurs technologiques de fabrication et des mécanismes régissant la libération du principe actif à partir de ces matrices en sont souvent restées aux stades fondamentaux sans parvenir à maîtriser l'ensemble de ces paramètres en vue d'élaborer une forme galénique qui puisse se concevoir industriellement. Ainsi, l'état de la technique antérieure peut être illustré par US-A-4 259 314 et US-A-4 261 970 ayant trait à la préparation de comprimés à libération contrôlée, notamment de théophylline. Les compositions tout comme les procédés décrits dans ces brevets ne permettent nullement une bonne maîtrise de la cinétique de libération du principe actif. Ils sont en particulier insusceptibles de conduire à une cinétique de libération d'ordre zéro.

Les connaissances sur la théophylline et les besoins des cliniciens font par exemple choisir un seul dosage unitaire de 300 mg (exprimé en anhydre) pour adulte.

Du fait de la posologie moyenne efficace ⩾ 600 mg/jour, il convient donc que la forme pharmaceutique assure une libération contrôlée du principe actif pendant environ 12 heures, ce qui correspond approximativement à la durée maximale de transit d'une forme galénique au niveau d'une zone d'absorption potentielle.

Les caractéristiques biogaléniques de la forme matrice hydrophile doivent permettre d'envisager une posologie journalière simple en deux prises par jour.

Du fait de la grande variété interindividuelle, il est toutefois nécessaire de pouvoir adapter la posologie. Pour faciliter cette adaptation, il est avantageux de concevoir une forme sécable. La caratéristique de sécabilité constitue l'une des originalités de la matrice hydrophile selon l'invention, puisqu'elle supprime ainsi l'utilisation simultanée de deux dosages unitaires (100 et 200 mg par exemple) ce qui est impossible avec toutes les formes pharmaceutiques du type microgranules ou matrices non homogènes.

Pour cela, avant même de pouvoir concevoir la sécabilité de la matrice, il convient de maîtriser parfaitement la cinétique de libération à partir de la surface globale du comprimé, après quoi seulement l'orientation vers une forme sécable pourra être envisagée. Il s'agira d'obtenir, par fractionnement, deux demi-matrices identiques entre elles et présentant des caractéristiques biogaléniques proches de la matrice entière.

Il apparaît souhaitable que le poids moyen du comprimé pour adulte soit avantageusement compris au maximum entre 500 et 550 mg.

La théophylline utilisée est par exemple une base monohydratée choisie sur des critères physicochimiques particuliers, la quantité utilisée équivalente à 300 mg de principe actif anhydre correspond à 330 mg de produit monohydraté. D'emblée il est possible de constater que le pourcentage de théophylline atteindra 60 à 66% du poids global du comprimé, ce qui constitue a priori un handicap majeur pour la mise au point de la formulation. En effet, dans ces conditions, la fraction disponible pour les excipients se trouve considérablement limitée.

Il a donc fallu opérer un choix judicieux sur le polymère hydrophile à utiliser, à la fois qualitativement et quantitativement. Pour mieux comprendre la mise au point du produit et le caractère inventif de l'application à la théophylline des matrices hydrophiles, il convient de bien sérier le processus de libération d'une substance médicamenteuse incorporée dans ce type de forme pharmaceutique. Il se subdivise en quatre stades essentiels:

*Stade 1:* pénétration du milieu de dissolution dans le comprimé avec libération simultanée d'une faible quantité de principe actif.

*Stade 2:* gonflement de la gomme hydrophile par absorption d'eau et formation d'une barrière gélifiée.

*Stade 3:* pénétration du liquide dans les zones profondes du comprimé par diffusion à travers la couche gélifiée et dissolution du principe actif.

*Stade 4:* diffusion en retour du principe actif dissous à travers la membrane gélifiée.

Les deux premiers stades constituent la phase d'hydratation qui est grandement influencée par les qualités intrinsèques du polymère, des adjuvants et du principe actif lui-même. Le comprimé mis en contact avec le milieu de dissolution va être rapidement mouillé et, de ce fait, assurer la stabilisation instantanée d'une faible quantité de principe actif.

La phase d'hydratation pendant laquelle s'établit le réseau hydrophile détermine la libération, cette période correspond à une modification profonde de la matrice.

Le polymère retenu est l'hydroxypropylméthylcellulose qui peut être utilisé sous différentes viscosités nominales. Il s'est avéré que cette dernière permet d'obtenir un bon réseau hydrophile et une gélification correcte sous certaines conditions et pour des concentrations particulières.

L'incorporation d'un agent liant est particulièrement délicate, en effet, la quantité de polymère hydrophile utilisée ne permet pas l'utilisation d'un agent inerte insoluble qui aura tendance à jouer le rôle de désintégrant condamnant ainsi l'utilisation de composés hautement liant comme les celluloses microcristallines.

Conformément à la présente invention, le choix s'est porté sur l'utilisation du lactose — en tant qu'agent liant hydrophile — lequel, tout en jouant ainsi le rôle de liant, favorise par son hydrophilie l'hydratation de la matrice et l'écoulement du mélange.

Il convient cependant de noter que le gonflement rapide de la matrice a tendance à lui donner un aspect pelucheux, et l'on assiste alors à une érosion qui perturbe la phase de diffusion.

La solution a été apportée par l'utilisation simultanée d'un agent lubrifiant interne hydrophobe. En effet, de façon à diminuer l'adhérence des poudres sur les poinçons, il a été adjoint au mélange par exemple du stéarate de magnésium. Il a ainsi été observé que le gonflement de la matrice fait apparaître un phénomène nouveau. L'hydratation est beaucoup plus lente du fait de la pellicule hydrophobe de stéarate de magnésium, et le comprimé subit alors une gélification progressive sans érosion et de façon homogène. La phase d'hydratation est alors maîtrisée et la quantité libérée à l'instant t = 1 h, soit $q_1$, peut être quantifiée précisément.

D'autre part, les particules de théophylline enrobées d'un film hydrophobe se dissolvent plus lentement et, le principe actif mis progressivement en solution à l'intérieur de la matrice va pouvoir diffuser selon la loi de Fick assurant une libération lente et constante. Cette seconde phase ou phase de diffusion stationnaire est caractérisée par la constante $k_r$; on obtient alors une cinétique d'ordre zéro caractérisant la forme pharmaceutique objet de la présente invention.

Conformément à la présente inention, le comprimé pharmaceutique constitué par une matrice polymère hydrophile permettant une libération contrôlée et constante d'une dose thérapeutique unitaire de théophylline pendant un intervalle de temps d'environ 12 heures, du type comprenant au moins un polymère dérivé de la cellulose et un agent lubrifiant, se caractérise en ce qu'il contient:

. une quantité thérapeutique efficace de théophylline;

. de 18% à 35% en poids par rapport au poids total du comprimé d'au moins une hydroxypropylméthylcellulose à titre de polymère hydrophile, la ou les hydroxypropylméthylcelluloses présentant une viscosité à 2% poids/poids à 20°C comprise entre $10^{-1}$ Pa·s et 15 Pa · s;

. de 7,5% à 22,5% en poids par rapport au poids total du comprimé de lactose favorisant l'hydratation du polymère, et

. de 0,5% à 1% en poids par rapport au poids total du comprimé d'un agent lubrifiant interne hydrophobe,

et en ce que le rapport de la théophylline — exprimé en poids anhydre — par rapport au polymère est compris entre 1 et 3.

Une large expérimentation pratique a permis de retenir un certain nombre d'autres caractéristiques additionnelles favorisant une bonne libération contrôlé de la théophylline.

C'est ainsi que le rapport pondéral optimal du polymère hydrophile par rapport au poids total du comprimé est avantageusement compris entre 20% et 26%.

Pour obtenir un comprimé pharmaceutique présentant une bonne stabilité et de bonnes propriétés de libération controlée, il est souhaitable d'utiliser une théophylline et une ou plusieurs hydroxypropylméthylcelluloses présentant des granulométries compatibles. La granulométrie de la théophylline, déterminée par ultra-sons, est par exemple avantageusement choisie de manière que 70% à 90% des particules de théophylline sont distribués entre 25 µm et 85 µm, avec une médiane en poids comprise entre 40 µm et 60 µm. La granulométrie de l'hydroxypropylméthylcellulose, déterminée par ultra-sons, est par exemple avantageusement choisie de telle manière que 65% à 85% des particules de polymère sont distribués entre 25 µm et 105 µm, avec une médiane en poids comprise entre 65 µm et 85 µm.

Tel que cela a déjà été exprimé précédemment, le polymère hydrophyle retenu conformément à la présente invention est une hydroxypropylméthylcellulose ou encore un mélange de plusieurs hydroxypropylméthylcelluloses de viscosités nominales différentes.

La nature et les quantités relatives de chaque constituant du comprimé objet de la présente invention ont également été soigneusement déterminées afin d'obtenir un pourcentage de théophylline libéré au cours de la première heure suivant l'ingestion qui est compris entre 10% et 20%, de préférence entre 10% et 15%.

Au cours des diverses expérimentations réalisées, il est apparu qu'un certain nombre de paramètres de mise en œuvre du procédé de fabrication des comprimés pouvaient également avoir une influence sur les propriétés physiques et thérapeutiques du comprimé de théophylline. Deux paramètres particuliers se sont avérés influencer de façon déterminante les propriétés du comprimé, à savoir la force de compression et le temps de mélange du stéarate de magnésium.

Il a donc tout d'abord été étudié l'influence de la force de compression sur un mélange réalisé en préformulation. Cette étude a eu pour but de déterminer la relation existant entre la force de compression et la résistance à l'écrasement, entre la force de compression et la quantité $q_1$ de principe actif libérés à l'instant t = 1 h, enfin entre la force de compression et la constante de vitesse de libération $k_r$.

Les conditions opératoires de cette expérimentation sont les suivantes:

mélange des matières premières sur mélangeur cubique:
- Théophylline (T)
- Hydroxypropylméthyl-
- cellulose(s) (HPMC)
- Lactose (L)
- Silice colloïdale (SC)

pendant 20 minutes.

Adjonction de stéarate de magnésium (SMg) et mélange 10 minutes.

Compression sur alternative Korsch équipée de jauges de contraintes-poinçons ronds de 1 cm² de surface.

Force de compression exprimée en Newtons (F).

Résistance à l'écrasement (RE) sur appareil Schleuniger mesurée en kp, 1 heure après la compression.

$q_1$ exprimée en % dissous à t = 1 h.

$k_r$ exprimée en % $h^{-1}$ (quantité dissoute/heure).

Les résultats observés ont été répertoriés dans le tableau I suivant:

### TABLEAU I

| F | RE | $q_1 \pm \sigma$ | $k_r$ |
|---|---|---|---|
| 7.500 | 6,83 | 20,54 ± 1,91 | 4,29 |
| 10.000 | 10,00 | 18,90 ± 0,97 | 4,49 |
| 12.500 | 12,27 | 17,21 ± 1,77 | 4,25 |
| 15.000 | 13,27 | 15,97 ± 2,07 | 4,28 |
| 17.500 | 14,97 | 16,01 ± 1,02 | 4,31 |
| 20.000 | 17,83 | 16,07 ± 2,10 | 4,18 |

Pour déterminer l'influence du temps de mélange du stéarate de magnésium on opère sur la formulation définitive avec des poinçons oblongs de 16 mm × 7 mm et une pression constante (~ 10.000 N). On mesure ainsi la quantité $q_1$ libérée à l'instant t = 1 h et on détermine la constante de vitesse de libération $k_r$ entre 1 heure et 8 heures.

Les résultats observés sont répertoriés dans le tableau II ci-sprès:

### TABLEAU II

| Conditions de mélange | temps en minutes | $q_1 \pm \sigma$ | $k_r$ |
|---|---|---|---|
| T + HPMC + L + SC + SMg | 20 10 | 20,45 ± 0,58 | 5,62 |
| T + HPMC + L + SC + SMg | 15 | 20,47 ± 2,54 | 5,64 |
| T + HPMC + L + SC + SMg | 30 | 16,89 ± 0,98 | 5,75 |

Ces différentes expérimentations ont amené à conclure:

1) que la pression n'influence pas la constante de vitesse de libération $(k_r)$ ce qui constitue le point remarquable de cette forme, la cinétique étant toujours d'ordre zéro entre 1 h et 8 h quelle que soit la force appliquée;

2) que la quantité $q_1$ libérée à t = 1 h diminue significativement lorsqu'on augmente F pour se stabiliser à partir de 15.000 Newtons;

3) que la résistance à l'écrasement croît parallèlement à l'augmentation de la force de compression, étant bien entendu que la relation qui s'en dégage ne s'applique qu'à un temps de mélange du stéarate de magnésium strictement défini, et

4) comme on pouvait s'y attendre on note le rôle important du temps de mélange du stéarate de magnésium avec les autres excipients et par conséquence sur la quantité libérée à t = 1 h; par contre, ce temps de mélange ne paraît pas influencer la constante de vitesse de libération $k_r$.

Si l'on rappelle l'un des buts initial de la présente invention, à savoir obtenir une valeur de $q_1$ comprise entre 10 et 20% et de préférence entre 10% et 15% de minimiser au maximum la fluctuation de théophyllinémie due à cette première phase, il convient donc de mieux contrôler cette phase d'hydratation de la matrice.

Pour diminuer la quantité $q_1$, deux solutions sont donc a priori envisageables:

— soit augmenter la force de compression F tout en conservant un temps de mélange de 10 ou 15 minutes; néanmoins, la force à appliquer plus importante risque d'entraîner des problèmes d'usure plus marqués de l'outillage à long therme;

— soit augmenter le temps de mélange du stéarate de magnésium, ce qui permet de diminuer notablement la quantité $q_1$. Cependant, il faudra veiller à ne pas perdre les propriétés de cohésivité du mélange, afin d'obtenir une résistance à l'écrasement satisfaisante du comprimé. Il a ainsi été déterminé qu'il convenait de ne pas dépasser un temps de mélange d'environ 30 minutes, particulièrement si l'on opère au mélangeur planétaire.

De plus, l'augmentation du temps de mélange entraîne une meilleure lubrification qui permet de diminuer la force d'éjection.

Au vu des résultats dégagés par les précédentes études, la possibilité de contrôler au mieux la phase d'hydratation de la matrice consiste à jouer à la fois sur le temps de mélange du stéarate de magnésium et sur la force de compression.

Conformément au procédé de fabrication industrielle selon la présente invention, on mélange l'ensemble des constituants pendant une période comprise entre 15 minutes et 30 minutes, et l'on réalise la mise en forme des comprimés par compression sous une pression moyenne de 3 tonnes à 7 tonnes, de préférence sous une force de compression environ égale à 5 tonnes.

Les conditions opératoires d'une fabrication industrielle peuvent par exemple être vérifiées de la manière suivante.

On mélange l'ensemble des excipients pendant environ 30 minutes et la mise en forme des comprimés est assurée par compression sur une pastilleuse rotative équipée de jauges de contraintes avec un outillage de poinçons oblongs de 16 mm × 7 mm. Les pressions lues ont été exprimées en tonnes. Les résultats ont été répertoriés dans le tableau III ci-après:

### TABLEAU III

| | F(T) | $q_1 \pm \sigma$ | $k_r$ |
|---|---|---|---|
| Lot A | 4 < F < 6 | 12,81 ± 0,52 | 5,09 |
| Lot B | | 12,07 ± 0,68 | 5,22 |
| Lot C | 7 < F < 8 | 11,36 ± 0,62 | |
| Lot D | 5 < F < 6 | 13,65 ± 0,48 | |

A titre d'exemple particulier de comprimé pharmaceutique conforme à la présente invention, on mentionnera la composition suivante:

| | |
|---|---|
| Théophylline monohydraté | 330 mg |
| Hydroxypropylméthylcellulose ($10^{-1}$ Pa·s) | 92 mg |
| Hydroxypropylméthylcellulose (15 Pa·s) | 25 mg |
| Lactose | 80 mg |
| Stéarate de magnésium | 5 mg |
| Silice colloïdale | 3 mg |
| Total | 535 |

Possédant une bonne maîtrise de la forme pharmaceutique, il s'est avéré judicieux de passer de la forme ronde classique et difficilement sécable, à une forme oblongue, par exemple 16 mm × 7 mm × 5 mm (L × l × e) avec barre de cassure sur la face supérieure. La forme oblongue présente l'avantage d'un fractionnement aisé, de plus la cassure très franche assure non seulement un poids équivalent des deux demi-comprimés mais, ce qui est remarquable, la libération de la théophylline à partir de ces deux demi-matrices conserve le profil de la cinétique du comprimé entier comme le témoignent les tests réalisés in vitro. L'étude in vivo réalisée sur 6 sujets adultes volontaires sains a montré des cinétiques plasmatiques superposables.

Le fait d'avoir utilisé un agent lubrifiant interne hydrophobe, ce qui peut paraître contradictoire au sein d'une matrice hydrophile constituée par ailleurs uniquement d'excipients hydrophiles, permet d'obtenir un meilleur contrôle de la phase d'hydratation de la matrice pendant laquelle s'établit le réseau hydrophile qui détermine la libération.

Cette période correspond à une modification profonde de la matrice en vue de l'établissement de la cinétique de libération, un gonflement homogène sans déformation notable, ni peluchage du comprimé durant cette phase d'hydratation conditionne la qualité de la libération durant la seconde phase du processus ou phase de diffusion stationnaire. L'utilisation de cet antagonisme hydrophilie-hydrophobicité, associé à une formulation judicieuse, permet une hydratation contrôlée des macromolécules au contact des fluides et donne naissance à une barrière gélifiée au travers de laquelle le principe actif, mis progressivement en solution à l'intérieur de la matrice, va pouvoir diffuser et assurer une libération lente et constante.

Dans le cas particulier des comprimés de théophylline, on obtient une forme pharmaceutique qui présente in vitro une cinétique de libération d'ordre zéro et qui permet, in vivo, de minimiser les fluctuations de la théophyllinémie tout en maintenant une thérapeutique efficace.

La mise au point d'une matrice homogène a également permis la réalisation d'une forme sécable. En effet, la sécabilité d'une forme à libération contrôlée n'est envisageable que dans la mesure où l'on obtient des demi-matrices qui présentent des paramètres biogaléniques identiques à ceux de la forme entière. Ceci est parfaitement réalisable dans le cas particulier des comprimés de théophylline selon l'invention qui présentent une cinétique de libération d'ordre zéro et une différence négligeable dans la constante de vitesse de libération $k_r$. Dans le cas des comprimés de l'invention, le parfait contrôle de l'hydratation et de la diffusion de la théophylline au sein de la trame gélifiée autorise le fractionnement de la matrice hydrophile. Il est donc possible d'obtenir deux fractions de comprimés qui conservent les paramètres biogaléniques de la matrice entière.

Après avoir maîtrisé, à la suite des études fondamentales, la libération in vitro de la théophylline à partir de la matrice selon un processus d'ordre zéro, les premiers résultats obtenus in vivo ont rapidement confirmé l'intérêt de cette forme pharmaceutique.

L'étude d'un lot pilote possédant in vitro une constante de libération ($k_r$) de 7,00 %·$h^{-1}$ avec un $q_1$ de 15% assure après dose unique de 300 mg chez deux volontaires sains une théophyllinémie proche de 5 µg·$ml^{-1}$ entre la 3ème heure et la 12ème heure. La simulation des taux sanguins à l'état d'équilibre permet d'envisager, pour ces deux sujets témoins et sur la base d'une posologie de 600 mg/jour une théophyllinémie à l'état d'équilibre voisine de 10 à 15 µg·$ml^{-1}$.

Au vu de ces résultats, la fabrication de deux lots A et B (réalisés à une semaine d'intervalle dans des conditions de fabrication industrielle) a été programmée.

*Les normes suivantes ont été établies:*

— La valeur de $q_1$ représentant le pourcentage libéré pendant la première heure a été fixée à 15% ± 5% compte tenu de la libération instantanée de la théophylline qui se trouve à la périphérie de la matrice.

$$10 \leqslant q_1\ (\%) \leqslant 20$$

— La valeur de $k_r$, constante de vitesse de libération, a été fixée à:

$$4,50 \leqslant k_r\ (\%\cdot h^{-1}) \leqslant 11,00$$

La prochaine étape a donc consisté à valider au plan galénique la forme pharmaceutique par l'étude et la comparaison in vitro des lots A et B par rapport au lot pilote. Ces lots correspondant à une composition particulière correspondant à l'exemple précis énoncé précédemment.

La validation galénique a ainsi permis de vérifier:

- la conformité des lots A et B

- la faible variabilité des paramètres de dissolution au sein d'un même lot

- la validité de l'équation linéaire $q = k_r t + b$, donc de type $y = ax + b$

- la secabilité de la matrice.

### CONFORMITE DES LOTS A ET B

Les études in vitro portant sur la cinétique de dissolution, détermination du pourcentage de théophylline libérée en fonction du temps en heure, sont mentionnées dans le tableau IV ci-après:

## TABLEAU IV

| Pourcentage dissous cumulé (n = 6) | | | | | | | |
|---|---|---|---|---|---|---|---|
| Lot A | $q_1$ | $q_2$ | $q_3$ | $q_4$ | $q_5$ | $q_6$ | $q_7$ | $q_8$ |
| m | 12,81 | 19,01 | 23,99 | 29,55 | 34,31 | 39,34 | 43,95 | 48,87 |
| s | 0,52 | 1,09 | 0,59 | 0,72 | 0,72 | 0,82 | 1,13 | 1,50 |
| CV | 4,05 | 5,7 | 2,45 | 2,45 | 2,10 | 2,10 | 2,60 | 3,10 |
| Lot B | $q_1$ | $q_2$ | $q_3$ | $q_4$ | $q_5$ | $q_6$ | $q_7$ | $q_8$ |
| m | 12,07 | 17,36 | 22,77 | 28.12 | 33,31 | 37,77 | 43,23 | 49,10 |
| s | 0,68 | 0,76 | 0,55 | 0,43 | 0,73 | 1,00 | 1,16 | 1,58 |
| CV | 5,65 | 4,40 | 2,40 | 1,55 | 2,20 | 2,65 | 2,70 | 3,20 |

m = moyenne  s = écart type  n = nombre
CV = coefficient de variation (pourcentage de l'écart type (s) par rapport à la valeur moyenne correspondante (m).

La linéarisation des cinétiques selon l'équation $q = k_r t + b$ fait apparaître:

pour le lot A . . . . . $q = 5.09t + 8.55$ $r^2 = 0.999$
pour le lot B . . . . . $q = 5.22t + 6.96$ $r^2 = 0.999$

Le respect des normes est donc bien assuré comme le montre le tableau V ci-dessus:

### TABLEAU V

| | Normes | n° lot Lot pilote | A | B |
|---|---|---|---|---|
| $q_1$ (%) | $10 \leqslant q_1 \leqslant 20$ | 15.00 | 12.81 | 12.07 |
| $k_r$ (% ·h⁻¹) | $4.50 \leqslant k_r \leqslant 11.00$ | 7.00 | 5.09 | 5.22 |

La faible variabilité des paramètres de dissolution entre les différentes lots permet d'apprécier la fiabilité et la reproductibilité de la technique de fabrication.

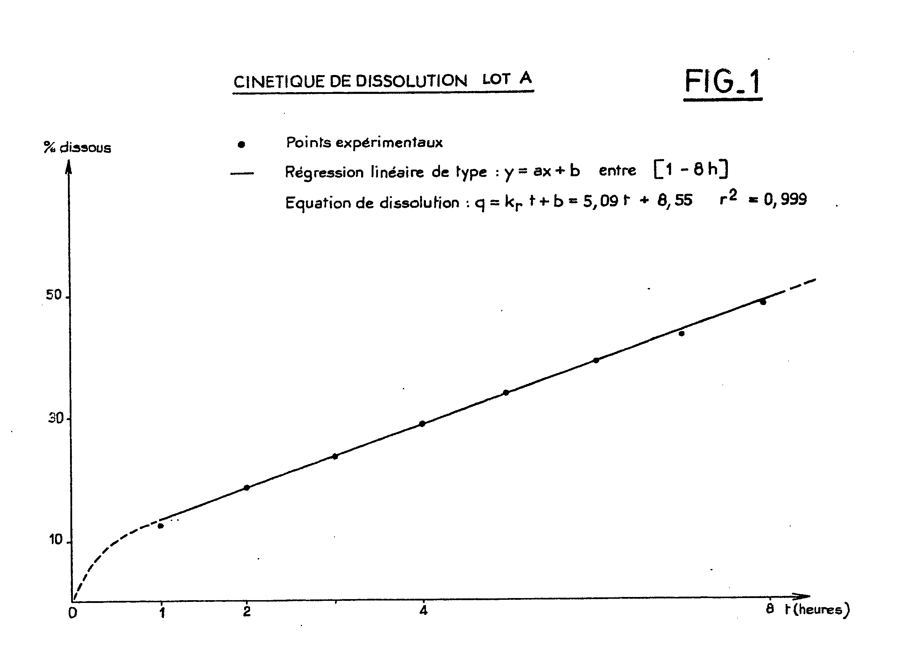

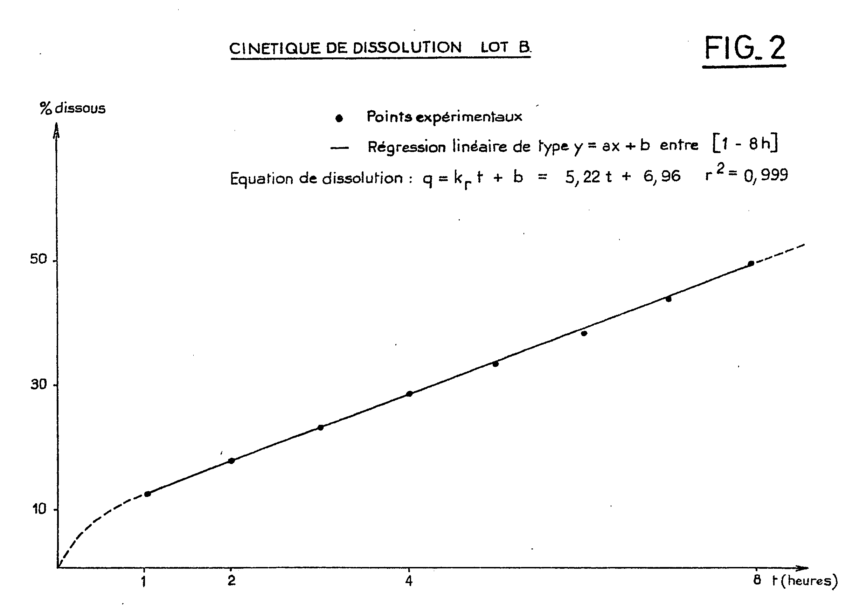

Les cinétiques de dissolution des lots A et B ont été présentées sur les figures 1 et 2 annexées.

### VARIABILITE DES PARAMETRES DE DISSOLUTION AU SEIN DU MEME LOT

Dans cette étude, nous avons décomposé la cinétique de dissolution de chacun des 6 comprimés qui ont servi au calcul de la cinétique de libération moyenne du lot A exprimée par l'équation:

$$q = k_r t + b = 5.09t + 8.55$$

et

$$q_1 = 12.81.$$

Les valeurs individuelles provenant des six comprimés sont référencées dans le tableau VI ci-dessous:

### TABLEAU VI

| | $q_1$ | $q = k_r t + b$ | $r^2$ |
|---|---|---|---|
| cpr 1 | 12.71 | $q = 5.05t + 9.03$ | 0.999 |
| cpr 2 | 13.34 | $q = 5.13t + 9.26$ | 0.998 |
| cpr 3 | 12.07 | $q = 4.98t + 8.04$ | 0.996 |
| cpr 4 | 12.39 | $q = 5.09t + 8.05$ | 0.999 |
| cpr 5 | 13.34 | $q = 5.36t + 8.22$ | 0.999 |
| cpr 6 | 13.01 | $q = 4.96t + 8.71$ | 0.998 |

La valeur moyenne de chaque paramètre avec écart type (s) et coefficient de variation (CV) sont présentés dans le tableau VII ci-après:

### TABLEAU VII

| | $q_1$ | $K_r$ |
|---|---|---|
| m | 12.81 | 5.09 |
| s | 0.52 | 0.14 |
| CV | 4.06 | 2.75 |

Le tableau VII permet de conclure que les coefficients de variation sont très faibles, et que la valeur moyenne de l'équation de dissolution d'un lot donné est bien en première approximation le reflet de la cinétique individuelle de tout comprimé pris au hasard dans ce lot.

### VALIDITE DE L'EQUATION LINEAIRE $q = k_r t + b$

La libération de la théophylline hors de la matrice hydrophile est optimisée par un modèle mathématique linéaire de type Y = ax + b, traduisant ainsi une cinétique d'ordre zéro.

Pour valider le modèle retenu, il a été montré qu'il n'existait pas de différence significative entre les valeurs expérimentales et les valeurs calculées à partir de l'équation moyenne du lot correspondant.

Les données concernant les lots A et B sont regroupées dans le tableau VIII ci-après:

TABLEAU VIII

| | | Pourcentage dissous cumulé (n = 6) | | | | | | | |
|---|---|---|---|---|---|---|---|---|---|
| | | $q_1$ | $q_2$ | $q_3$ | $q_4$ | $q_5$ | $q_6$ | $q_7$ | $q_8$ |
| lot A | valeurs expérimentales | 12,81 | 19,01 | 23,99 | 29,55 | 34,31 | 39,34 | 43,95 | 48,87 |
| | valeurs calculées (q = 5.09t + 8.55) | 13,64 | 18,73 | 23,82 | 28,91 | 34,00 | 39,09 | 44,18 | 49,27 |
| lot B | valeurs expérimentales | 12,07 | 17,32 | 22,77 | 28.12 | 33,31 | 37,77 | 43,23 | 49,10 |
| | valeurs calculées (q = 5.22t + 6.96) | 12,18 | 17,40 | 22,62 | 27,84 | 33,06 | 38,28 | 43,50 | 48,72 |

*Test statistique*

Après avoir vérifié la normalité de la distribution, un test de t pour série appariée de faible effectif a été effectué pour chacun des lots, il en résulte:

Lot A . . . . . t = 0,142  ddl = 7  NS
Lot B . . . . . t = 0,104  ddl = 7  NS

Ces résultats montrent que pour un nombre de ddl de 7 et le risque de 5%, il n'y a pas de différence significative entre valeurs expérimentales et valeurs calculées à partir de la droite de type y = ax + b, ce qui valide parfaitement le modèle retenu pour optimiser la cinétique de libération de la théophylline à partir de la forme pharmaceutique.

*Expression du flux de libération en mg · h⁻¹*

L'expression q = $k_r$t + b permet bien évidemment de calculer la quantité de théophylline, exprimée en mg, et libérée à chaque instant t; on parlera alors de flux de libération.

La constante $k_r$ (% · h⁻¹) représente en effet un pourcentage de théophylline libérée par heure, exprimée par rapport à la dose théorique présente dans une matrice soit 300 mg = 100%; ainsi le flux de libération «F» pour un $k_r$ moyen de 7,00 % · h⁻¹ est égal à 21,00 mg · h⁻¹ (1% = 3 mg).

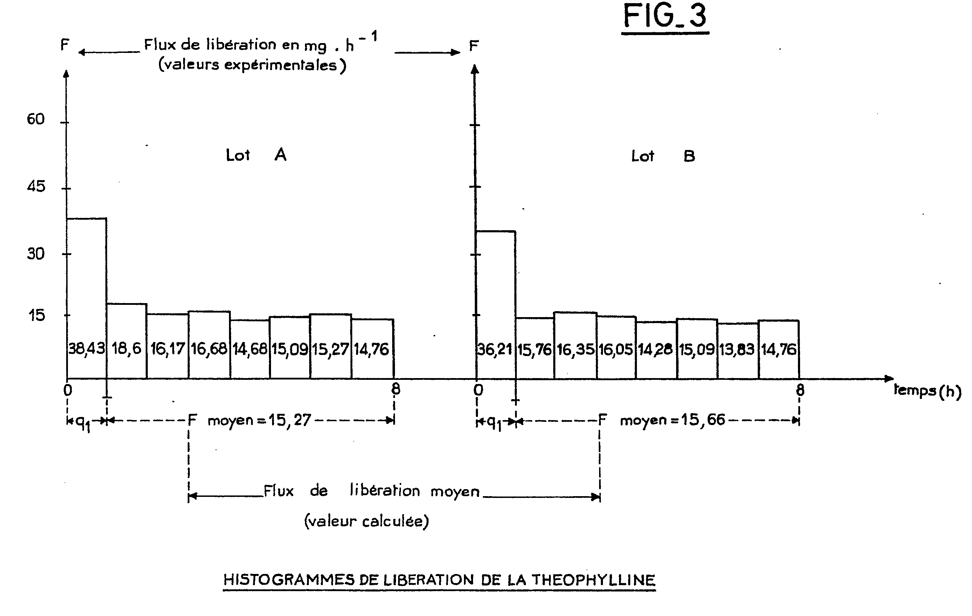

Les lots A et B, dont les histogrammes sont représentés à la figure 3 annexée possèdent un flux respectif moyen de 15.27 et 15.66 mg · h⁻¹.

*SECABILITE DE LA MATRICE HYDROPHILE*

*Validation par pesée unitaire des demi-comprimés*

La validation de la bonne sécabilité de la matrice est réalisée à partir de 10 comprimés pris au hasard, répertoriés P¹ à P¹⁰ et fractionnés en deux. Les demis provenant d'un même comprimé sont indiqués:

$P_a^1$ , $P_b^1$ . . . . . $P_a^{10}$, $P_b^{10}$

et pesés. Leurs poids respectifs en grammes sont référencés dans le tableau IX ci-dessous:

TABLEAU IX

| comprimés | | 1 | 2 | 3 | 4 | 5 |
|---|---|---|---|---|---|---|
| demi-comprimés | $P_a$ | 0,2684 | 0,2599 | 0,2605 | 0,2662 | 0,2747 |
| | $P_b$ | 0,2632 | 0,2692 | 0,2705 | 0,2659 | 0,2577 |
| comprimés | | 6 | 7 | 8 | 9 | 10 |
| demi-comprimés | $P_a$ | 0,2692 | 0,2655 | 0,2511 | 0,2699 | 0,2646 |
| | $P_b$ | 0,2617 | 0,2645 | 0,2769 | 0,2615 | 0,2672 |

*Analyse statistique*

Comparaison pour chaque comprimé de $P_a/P_b$ par un test de t pour séries appariées.

TABLEAU X

|  | moyenne | écart-type | t | ddl | signification | CV |
|---|---|---|---|---|---|---|
| $P_a$ | 0,2650 | 0,0066 |  |  |  | 2,49 |
|  |  |  | 0,219 | 9 | NS |  |
| $P_b$ | 0,2658 | 0,0055 |  |  |  | 2,07 |

En conclusion, il apparaît que les différences de poids entre les fractions a et b de chaque comprimé ne sont pas statistiquement significatives. L'obtention de demi-comprimés d'un poids unitaire pratiquement équivalent ($CV < 5\%$) permet ainsi une adaptation précise de la posologie.

*Validation de la cinétique de libération de deux demi-comprimés*

Cette étude a eu pour but de comparer la cinétique de dissolution-comprimé entier/comprimé fractionné.

Les résultats obtenus sur le lot B sont mentionnés ci-après:

TABLEAU XI

|  | Pourcentage dissous cumulé (n = 6) | | | | | | | |
|---|---|---|---|---|---|---|---|---|
|  | $q_1$ | $q_2$ | $q_3$ | $q_4$ | $q_5$ | $q_6$ | $q_7$ | $q_8$ |
| m | 16,60 | 24,71 | 31,61 | 40,37 | 47,75 | 54,75 | 62,24 | 74,69 |
| s | 0,58 | 0,73 | 0,77 | 0,79 | 1,30 | 0,85 | 2,17 | 1,75 |
| CV | 3,49 | 2,95 | 2,44 | 1,96 | 2,72 | 1,55 | 3,38 | 2,34 |

*Equation de dissolution:* $\quad q = 8{,}11t + 7{,}85$
$$r^2 = 0{,}997$$

*Paramètres de dissolution:* $\quad q_1 = 16{,}60$
$$k_r = 8{,}11$$

**Analyse**

Le profil de dissolution reste inchangé (cinétique d'ordre O). La quantité $q_1$ libérée pendant la première heure est conforme aux normes mentionnées. La vitesse de libération est légèrement augmentée car elle est fonction de la surface de diffusion S de la matrice qui, dans ce cas, est précisément augmentée de deux sections. En résumé, on n'observe pas de modification profonde de la cinétique de libération de la théophylline, ce qui est dû à la présence d'un réseau structurel homogène constituant la matrice et qui autorise ainsi la sécabilité de la forme pharmaceutique.

Les caractéristiques pharmacocinétiques des comprimés selon l'invention fractionnés en deux éléments ont été comparées à celles des comprimés entiers. Cette expérimentation a été réalisée sur six sujets tirés au sort qui ont reçu un comprimé coupé en deux.

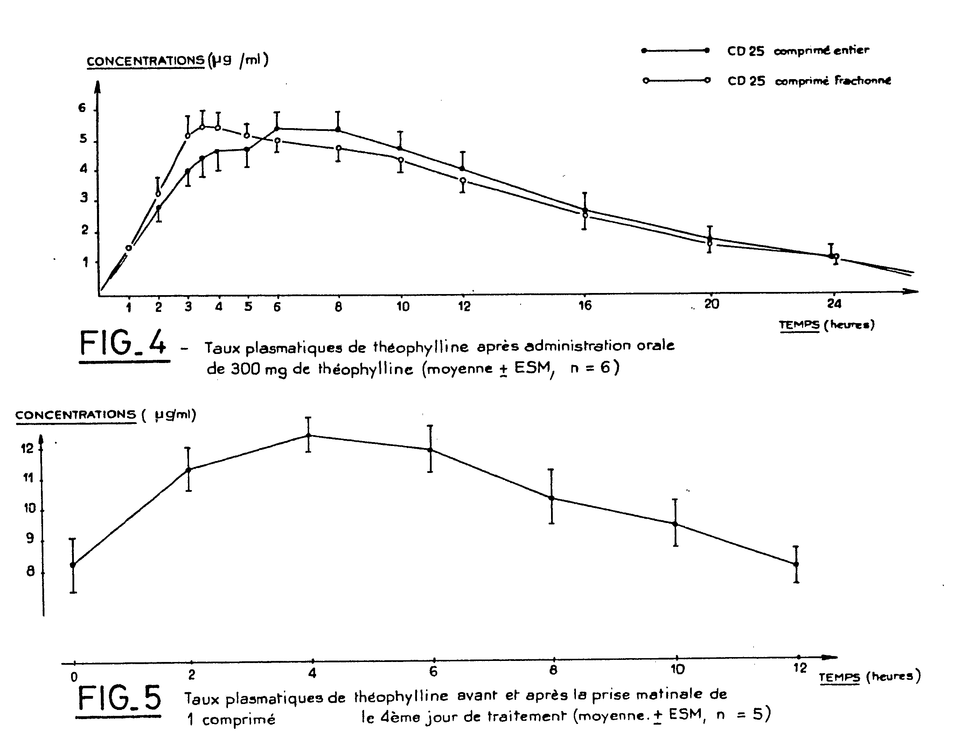

Les courbes moyennes de taux plasmatiques de théophylline sont représentées sur la figure 4 annexée.

A l'exception d'une faible accélération de la vitesse d'absorption due à l'augmentation de la vitesse de libération comme démontré in vitro, il n'a pas été noté de modification de la quantité totale absorbée ni des concentrations maximales obtenues. On peut donc assurer que l'adaptation posologique peut être réalisée avec les comprimés selon l'invention de manière adéquate par demi-comprimé, sans modifier les caractéristiques de la cinétique plasmatique de le théophylline.

Enfin, un essai d'administration répétée de comprimés selon l'invention a permis de vérifier l'obtention de l'état d'équilibre après administrations réitérées de deux comprimés par jour durant 4 jours. Pour cela six sujets ont reçu un comprimé selon l'invention matin et soir, soit 600 mg de théophylline anhydre par jour (en fait 5 sujets seulement ont terminé l'étude, le 6ème ayant présenté une sensibilité particulière à la théophylline justifiant son retrait de l'essai).

La courbe moyenne illustrant les taux plasmatiques de théophylline avant et après la prise matinale d'un comprimé le 4ème jour de traitement est présentée à la figure 5 annexée.

A l'examen du diagramme de la figure 5, on constate que l'état d'équilibre est bien atteint en 4 jours d'administration. De plus, en utilisant un programme de simulation, il a été démontré que cet état d'équilibre était en fait obtenu dès la fin du 2ème jour d'administration.

**Revendications**

1. Comprimé pharmaceutique constitué par une matrice polymère hydrophile permettant une libération contrôlée et constante d'une dose thérapeutique unitaire de théophylline pendant un intervalle de temps d'environ 12 heures, du type comprenant au moins un polymère dérivé de la cellulose et un agent lubrifiant, caractérise en ce qu'il contient:

. une quantité thérapeutique efficace de théophylline;

. de 18% à 35% en poids par rapport au poids total du comprimé d'au moins une hydroxypropylméthylcellulose à titre de polymère hydrophile, la ou les hydroxypropylméthylcelluloses présentant une viscosité à 2% poids/poids à 20°C comprise entre $10^{-1}$ Pa·s et 15 Pa·s;

. de 7,5% à 22,5% en poids par rapport au poids total du comprimé de lactose favorisant l'hydratation du polymère, et

. de 0,5% à 1% en poids par rapport au poids total du comprimé d'un agent lubrifiant interne hydrophobe,

et en ce que le rapport pondéral de la théophylline — exprimé en poids anhydre — par rapport au polymère est compris entre 1 et 3.

2. Comprimé selon la revendication 1, caractérisé en ce que le rapport pondéral du polymère hydrophile par rapport au poids total du comprimé est compris entre 20% et 26%.

3. Comprimé selon l'une des revendications 1 et 2, caractérisé en ce qu'il contient de l'ordre de 300 mg de théophylline anhydre.

4. Comprimé selon l'une des revendications 1 à 3, caractérisé en ce que la théophylline et l'hydroxypropylméthylcellulose utilisées présentent des granulométries compatibles.

5. Comprimé selon l'une des revendications 1 à 4, caractérisé en ce que la granulométrie de la théophylline, déterminée par ultra-sons, est telle que 70% à 90% des particules sont distribués entre 25 μm et 85 μm, avec une médiane en poids comprise entre 40 μm et 60 μm.

6. Comprimé selon l'une des revendications 1 à 5, caractérisé en ce que la granulométrie de l'hydroxypropylméthylcellulose déterminée par ultra-sons est telle que 65% à 85% des particules de polymère sont distribués entre 25 μm et 105 μm, avec une médiane en poids comprise entre 65 μm et 85 μm.

7. Comprimé selon l'une des revendications 1 à 6, caractérisé en ce que le polymère hydrophile est une hydroxypropylméthylcellulose ou un mélange de plusieurs hydroxypropylméthylcelluloses de viscosités nominales différentes.

8. Comprimé selon l'une des revendications 1 à 7, caractérisé en ce que l'agent lubrifiant interne hydrophobe est le stéarate de magnésium.

9. Comprimé selon l'une des revendications 1 à 8, caractérisé en ce que le pourcentage de théophylline libéré au cours de la première heure suivant l'ingestion est compris entre 10% et 20%.

10. Comprimé selon la revendication 9, caractérisé en ce que le pourcentage de théophylline libéré au cours de la première heure suivant l'ingestion est compris entre 10% et 15%.

11. Comprimé selon l'une des revendications 1 à 10, caractérisé en ce qu'il est sécable et qu'il présente de préférence une forme oblongue.

12. Comprimé selon l'une des revendications 1 à 11, caractérisé en ce qu'il présente la composition suivante:

| | |
|---|---:|
| Théophylline monohydraté | 330 mg |
| Hydroxypropylméthylcellulose ($10^{-1}$ Pa·s) | 92 mg |
| Hydroxypropylméthylcellulose (15 Pa·s) | 25 mg |
| Lactose | 80 mg |
| Stéarate de magnésium | 5 mg |
| Silice colloïdale | 3 mg |
| Total | 535 |

13. Procédé de fabrication d'un comprimé selon l'une des revendications 1 à 12, caractérisé en ce que l'on mélange l'ensemble des constituants pendant une période comprise entre 15 minutes et 30 minutes, puis que l'on réalise la mise en forme des comprimés par compression sous une pression moyenne de 3 tonnes à 7 tonnes.

14. Procédé selon la revendications 13, caractérisé en ce que la force de compression est environ égale à 5 tonnes.

15. Procédé selon l'une des revendications 13 et 14, caractérisé en ce que la mise en forme des comprimés est réalisée à l'aide d'une pastilleuse rotative équipée de poinçons de section oblongue.


**Patentansprüche**

1. Pharmazeutische Tablette bestehend aus einer Matrix aus einem hydrophilen Polymer, die eine gesteuerte und konstante Freisetzung einer therapeutischen Normaldosis Theophyllin während eines Zeitraumes von ungefähr 12 Stunden ermöglicht, jener Art mit mindestens einem von Zellulose abgeleiteten Polymer und einem Gleitmittel, dadurch gekennzeichnet, dass sie enthält:

. eine wirksame therapeutische Menge Theophyllin:

. 18 bis 35 Gew.-% im Verhältnis zum Gesamtgewicht der Tablette, mindestens einer Hydroxypropylmethylzellulose als hydrophiles Polymer, wobei die Hydroxypropylmethylzellulose oder -zellulosen eine Viskosität zwischen $10^{-1}$ Pa.s und 15 Pa.s bei 2 Gew.-% und 20°C aufweisen;

. 7,5 bis 22,5 Gew.-%, im Verhältnis zum Gesamtgewicht der Tablette, Lactose, die die Hydratation des Polymers begünstigt, und

. 0,5 bis 1 Gew.-%, im Verhältnis zum Gesamtgewicht der Tablette, eines internen hydrophoben Gleitmittels,

und dass der Gewichtsanteil von Theophyllin — wasserfrei — im Verhältnis zum Polymer zwischen 1 und 3 liegt.

2. Tablette nach Anspruch 1, dadurch gekennzeichnet, dass der Gewichtsanteil des hydrophilen Polymers im Verhältnis zum Gesamtgewicht der Tablette zwischen 20% und 26% liegt.

3. Tablette nach einem der Ansprüche 1 und 2, dadurch gekennzeichnet, dass sie Theophyllin — wasserfrei — in der Grössenordnung von 300 mg enthält.

4. Tablette nach einem der Ansprüche 1 bis 3, dadurch gekennzeichnet, dass das verwendete Theophyllin und die verwendete Hydroxypropylzellulose eine kompatible Korngrösse aufweisen.

5. Tablette nach einem der Ansprüche 1 bis 4, dadurch gekennzeichnet, dass die Korngrösse des Theophyllins, bestimmt durch Ultraschall, so ist, dass 70% bis 90% der Teilchen zwischen 25 µm und 85µm liegen, mit einem Mittelwert zwischen 40 µm und 60 µm.

6. Tablette nach einem der Ansprüche 1 bis 5, dadurch gekennzeichnet, dass die Korngrösse der Hydroxypropylmethylzellulose, bestimmt durch Ultraschall, so ist, dass 65% bis 85% der Polymerteilchen zwischen 25 µm und 105 µm liegen, mit einem Mittelwert zwischen 65 µm und 85 µm.

7. Tablette nach einem der Ansprüche 1 bis 6, dadurch gekennzeichnet, dass das hydrophile Polymer eine Hydroxypropylmethylzellulose oder eine Mischung aus mehreren Hydroxypropylmethylzellulosen unterschiedlicher Nominalviskosität ist.

8. Tablette nach einem der Ansprüche 1 bis 7, dadurch gekennzeichnet, dass das interne hydrophobe Gleitmittel Magnesiumstearat ist.

9. Tablette nach einem der Ansprüche 1 bis 8, dadurch gekennzeichnet, dass der Prozentsatz des im Laufe der ersten Stunde nach der Verdauung freigesetzten Theophyllins zwischen 10% und 20% liegt.

10. Tablette nach Anspruch 9, dadurch gekennzeichnet, dass der Prozentsatz des im Laufe der ersten Stunde nach der Verdauung freigesetzten Theophyllins zwischen 10% und 15% liegt.

11. Tablette nach einem der Ansprüche 1 bis 10, dadurch gekennzeichnet, dass sie teilbar ist und vorzugsweise eine längliche Form hat.

12. Tablette nach einem der Ansprüche 1 bis 11, dadurch gekennzeichnet, dass sie folgende Zusammensetzung aufweist:

| | |
|---|---|
| Monohydrogentheophyllin | 330 mg |
| Hydroxypropylméthylzellulose ($10^{-1}$ Pa·s) | 92 mg |
| Hydroxypropylméthylzellulose (15 Pa·s) | 25 mg |
| Lactose | 80 mg |
| Magnesiumstearat | 5 mg |
| Silika kolloidal | 3 mg |
| Insgesamt | 535 |

13. Verfahren zur Herstellung einer Tablette nach einem der Ansprüche 1 bis 22, dadurch gekennzeichnet, dass man die Gesamtheit der Bestandteile während eines Zeitraumes von 15 bis 30 Minuten mischt und dann das Formen der Tabletten durch Pressen unter einem mittleren Druck von 3 Tonnen bis 7 Tonnen durchführt.

14. Verfahren nach Anspruch 13, dadurch gekennzeichnet, dass die Presskraft ungefähr 5 Tonnen beträgt.

15. Verfahren nach einem der Ansprüche 13 und 14, dadurch gekennzeichnet, dass das Formen der Tabletten mit Hilfe einer rotierenden Tablettenmaschine erfolgt, die mit einem Stempel mit länglichem Querschnitt versehen ist.

**Claims**

1. Pharmaceutical tablet constituted by a hydrophilic polymeric matrix permitting controlled and constant release of a unitary therapeutic dose of theophyllin during a period of approximately 12 hours, of the type comprising at least one polymer derived from cellulose and a lubricating agent, characterised in that it contains:

. an effective therapeutic quantity of theophyllin;

. from 18% to 35% by weight as a ratio of the total weight of the tablet of at least one hydroxypropylmethylcellulose by way of hydrophyllic polymer, the hydroxypropylmethylcellulose(s) having a viscosity of 2% weight for weight at 20°C between $10^{-1}$ $P_a$s and 15 $P_a$.s;

. from 7.5% to 22.5% by weight as a ratio of the total weight of the tablet of lactose favouring hydration of the polymer, and

. 0.5% to 1% by weight as a ratio of the total weight of the tablet of an internal hydrophobic lubricating agent,

and in that the weight ratio of the theophyllin expressed in anhydrous weight as a ratio to the polymer is between 1 and 3.

2. Tablet according to claim 1, characterised in that the weight ratio of the hydrophyllic polymer as a ratio to the total weight of the tablet is between 20% and 26%.

3. Tablet according to claim 1 or claim 2, characterised in that it contains of the order of 300 mg of anhydrous theophyllin.

4. Tablet according to any one of claims 1 to 3, characterised in that the theophyllin and the hydroxypropylmethylcellulose utilised have compatible granulometries.

5. Tablet according to any one of claims 1 to 4, characterised in that the granulometry of the theophyllin, determined by ultrasound, is such that 70% to 90% of its particles are distributed between 25 µm and 85 µm, with a weighted median between 40 µm and 60 µm.

6. Tablet according to any one of claims 1 to 5, characterised in that the granulometry of the hydroxypropylmethylcellulose determined by ultrasound is such that 65% to 85% of the particles of polymer are distributed between 25 µm and 105 µm, with a weighted median between 65 µm and 85 µm.

7. Tablet according to any one of claims 1 to 6, characterised in that the hydrophyllic polymer is a hydroxypropylmethylcellulose or a mixture of several hydroxypropylmethylcelluloses of different nominal viscosities.

8. Tablet according to any one of claims 1 to 7, characterised in that the hydrophobic internal lubricanting agent is magnesium stearate.

9. Tablet according to any one of claims 1 to 8, characterised in that the percentage of theophyllin freed in the course of the first hour following ingestion is between 10% and 20%.

10. Tablet according to claim 9, characterised in that the percentage of theophyllin freed in the course of the first hour following ingestion is between 10% and 15%.

11. Tablet according to any one of claims 1 to 10, characterised in that it is divisible and preferably has an oblong form.

12. Tablet according to any one of claims 1 to 11, characterised in that it has the following composition:

| | |
|---|---:|
| Monohydrated theophyllin | 330 mg |
| Hydroxypropylmethylcellulose ($10^{-1}$ $P_a \cdot s$) | 92 mg |
| Hydroxypropylmethylcellulose (15 $P_a \cdot s$) | 25 mg |
| Lactose | 80 mg |
| Magnesium stearate | 5 mg |
| Colloidal silicondioxide | 3 mg |
| Total | 535 |

13. Process for producing a tablet according to any one of claims 1 to 12, characterised in that the assembly of constituents are mixed for between 15 and 30 minutes, then they are formed into tablets by compression under an average pressure of 3 to 7 tonnes.

14. Process according to claim 13, characterised in that the compression force is approximately equal to 5 tonnes.

15. Process according to claim 13 or claim 14, characterised in that the forming into tablets is carried out with a rotary pelletiser equipped with oblong cross-section dies.

CINETIQUE DE DISSOLUTION  LOT  A          FIG.1

- Points expérimentaux
— Régression linéaire de type : $y = ax + b$   entre   $[1 - 8\,h]$
   Equation de dissolution : $q = k_r\,t + b = 5,09\,t + 8,55$   $r^2 = 0,999$

CINETIQUE DE DISSOLUTION   LOT B.

FIG. 2

% dissous

● Points expérimentaux

— Régression linéaire de type y = ax + b  entre [1 - 8 h]

Equation de dissolution : $q = k_r \, t + b = 5,22 \, t + 6,96$    $r^2 = 0,999$

## FIG_3

Flux de libération en mg . h$^{-1}$
(valeurs expérimentales)

Lot A

Lot B

38,43 | 18,6 | 16,17 | 16,68 | 14,68 | 15,09 | 15,27 | 14,76

36,21 | 15,76 | 16,35 | 16,05 | 14,28 | 15,09 | 13,83 | 14,76

temps (h)

$q_1$  F moyen = 15,27

$q_1$  F moyen = 15,66

Flux de libération moyen
(valeur calculée)

HISTOGRAMMES DE LIBERATION DE LA THEOPHYLLINE

0 109 320

FIG_4 — Taux plasmatiques de théophylline après administration orale de 300 mg de théophylline (moyenne ± ESM, n = 6)

Légende:
- CD 25 comprimé entier
- CD 25 comprimé fractionné

FIG_5 Taux plasmatiques de théophylline avant et après la prise matinale de 1 comprimé le 4ème jour de traitement (moyenne ± ESM, n = 5)

0 109 320